# EUROPEAN PATENT SPECIFICATION

(11) **EP 3 998 929 B1**
(45) Date of publication and mention of the grant of the patent: **08.10.2025**
(21) Application number: 20751426.6
(22) Date of filing: 14.07.2020
(51) Int. Cl.: A61B 1/00

(54) **ENDOSCOPIC TOOL STABILIZATION**
STABILISIERUNG VON ENDOSKOPISCHEM WERKZEUG
OUTIL DE STABILISATION ENDOSCOPIQUE

(30) Priority: 15.07.2019 US 201962874242 P
(43) Date of publication of application: 25.05.2022
(60) Divisional application: 25199736.7
(73) Proprietor: Boston Scientific Scimed, Inc., Maple Grove, MN 55311-1566 (US)
(72) Inventor: WILDER, Evan, Boston, MA 02135 (US); BRECHBIEL, Scott, E., Acton, MA 01720 (US); WELDON, James, Newton, MA 02459 (US)
(74) Representative: Vossius & Partner Patentanwälte Rechtsanwälte mbB
(86) International application number: PCT/US2020/041890
(87) International publication number: WO 2021/011517

(56) References cited:
- JP-A- 2012 070 792
- JP-U- H0 324 103
- JP-U- S 508 784
- JP-U- S5 020 489
- US-A- 4 436 087
- US-A1- 2019 059 702
- US-B1- 6 458 074

## Description

### TECHNICAL FIELD

Various aspects of the present disclosure relate generally to endoscopic devices. More specifically, the present disclosure relates to device tips for endoscopic tool stabilization and related methods of use.

### BACKGROUND

During both diagnostic and therapeutic endoscopic procedures, accessory devices may be passed through the working channel of an endoscope. The outer diameter of the accessory device should be compatible with the inner diameter of the working channel. Endoscopes used solely for diagnostic procedures generally have smaller working channels, compared to those used for combination (diagnostic and therapeutic) or solely therapeutic procedures. For example, diagnostic and therapeutic gastroscopes typically have working channel inner diameters of 2.8 mm and 3.7 mm, respectively. Accessory devices designed for use in diagnostic scopes are generally compatible with therapeutic scopes as well. However, accessory devices designed for use in diagnostic scopes may be undersized when used with therapeutic scopes, resulting in a loose fit within the working channel. Examples of endoscopic devices of the above-describe type are to be found in US 4436087 A, JP H03 24103 U, JP 2012 070792 A, JP S50 20489 U, US 6458074 B1, JP S50 8784 U and US 2019/059702. Said prior art documents disclose devices comprising (i) a shaft having a lumen terminating in a distally-facing opening, and (ii) an elevator including an actuator and a body configured to extend within the lumen for selectively positioning the instrument.

This loose fit can lead to accessory device instability as the scope articulates throughout the procedure. Accessory device instability during the procedure may result in variable orientation of the device within the working channel, as seen under direct visualization. Though device instability may not be problematic during some procedures, it can be an issue during more precise procedures (such as, e.g., endoluminal surgery). During endoluminal surgical procedures, a cutting knife may be used to excise tissue. Some existing cutting knives have no articulation capability, and cutting motions performed by the physician are controlled by articulation of the scope. In cases where the cutting knife is undersized relative to the inner diameter of the working channel of the endoscope, there is a loose fit between the knife and the working channel, and thus, the knife may move unexpectedly as the physician articulates the scope. This introduces a level of unpredictability for the physician performing the procedure and potential risk for the patient.

### SUMMARY

Embodiments of the present disclosure relate to, among other things, mechanisms for stabilizing a medical tool within a scope or like device. Each of the embodiments disclosed herein may include one or more of the features described in connection with any of the other disclosed embodiments. The invention is defined by the appended claims.

Devices according to the invention include a shaft having a distal end and a lumen, the lumen terminating in a distally-facing opening. An instrument may be inserted through the shaft and may extend through the lumen and out of opening. The devices of the invention further include an elevator for engaging the instrument. The elevator includes an actuator extending through at least a portion of the shaft; and a body coupled to the actuator. A portion of the body is configured to extend within the lumen for selectively positioning the instrument.

According to the invention as defined in independent claim 1, the body includes a first extension, a second extension, and a proximal portion connecting the first extension and the second extension. The actuator is coupled to a distal portion of the first extension and a portion of the second extension is configured to move within the lumen when the actuator is moved proximally. The portion of the second extension is configured to move out of the lumen when the actuator is moved distally. A longitudinal axis of the first extension may be transverse to a longitudinal axis of the second extension. The second extension has a U-shaped body surface for engaging the instrument. The body may rotate about an axis positioned within the proximal portion when the actuator is moved proximally or distally. According to the invention as defined in independent claim 4, the body includes a first extension, a second extension, a proximal portion connecting the first extension and the second extension, and a pivot member. A radially inner surface of the first extension and a radially inner surface of the second extension are configured to align with a radially inner surface of the lumen. The actuator is coupled to the pivot member and contacts a curved surface of pivot member. A portion of the first extension is configured to move within the lumen when the actuator is moved proximally; and a portion of the second extension is configured to move within the lumen when the actuator is moved distally.

According to the invention as defined in independent claim 6, the body is positioned within a channel extending distally from an opening in a radially inner surface of the lumen. The channel has a longitudinal axis transverse to the longitudinal axis of the lumen. A surface of the body is configured to slidably interface with the channel when the actuator is moved proximally or distally. The body may be configured to slide proximally within the channel, move through the opening in the radially inner surface of the lumen, and enter the channel, when the actuator is moved proximally. According to the invention as defined in independent claim 8, the elevator further includes a tab member pivotally movable relative to lumen; and a block fixedly coupled to the shaft and including a first surface contacting a second surface of the body. The first surface is transverse to the longitudinal axis of the lumen; and the second surface is configured to slidably interface with the first surface when the actuator is moved proximally or distally. The body is configured to move towards the lumen and force the tab member into the lumen. The tab member may be biased away from the lumen. The actuator may extend through a channel within the block. According to the invention defined in independent claim 11, the lumen is a first lumen, and the body is a snare loop. The elevator further includes a support including a second lumen and a channel extending circumferentially around a radially inner surface of the second lumen. The second lumen aligns with the first lumen. The channel receives the snare loop; and the snare loop is configured to enter the second lumen when the actuator is moved proximally.

In other aspects, a device may include a shaft having a distal end and a first lumen, the first lumen terminating in a distal-facing opening, wherein an instrument inserted through the shaft may extend through the lumen and out of the opening. The device may further include an elevator for engaging the instrument. The elevator may include a first actuator extending through at least a portion of the shaft and a rotatable plate coupled to the first actuator. The rotatable plate may be angled such that a distal portion of the rotatable plate is more distal relative to a proximal portion of the rotatable plate. The rotatable plate may include a second lumen configured to align with first lumen, and a recess. The elevator may further include a second actuator extending through at least a portion of the shaft and a slide member coupled to the second actuator. The slide member may be positioned within the recess and may include a third lumen configured to align with the first lumen. The elevator may also include a frame fixedly positioned within the shaft. The rotatable plate may be rotatably coupled to the frame.

In other aspects, the device may include one or more of the features below. A portion of the slide member may be configured to extend within the first lumen and to apply a force to the instrument when the second actuator is moved. The slide member may be configured to move the instrument such that the instrument contacts a radially inner surface of first lumen. The rotatable plate may be configured to rotate relative to the frame when the first actuator is moved, and rotation of the rotatable plate may rotate the slide member. The frame may include a wall, and the wall may be configured to limit the movement of the slide member within the recess of the rotatable plate.

In other aspects, not falling within the scope of the claimed subject-matter, a device may include a shaft having a distal end and a lumen terminating in a distally-facing opening, wherein an instrument inserted through the shaft may extend through the lumen and out of the opening, and an elevator for engaging the instrument. The elevator may include an actuator extending through the shaft; and a body coupled to the actuator and including an opening. The body may be configured to be positioned distally of the opening of the shaft and to apply a force to the instrument when the actuator is rotated about a longitudinal axis of the actuator The body may be configured to move the instrument such that the instrument contacts a radially inner surface of the lumen.

In other aspects, the device may include one or more of the features below. The opening of the body may be configured to align with the lumen, and a biasing member may be coupled to the body and may bias the body towards a position in which the opening of the body is aligned with the lumen.

It may be understood that both the foregoing general description and the following detailed description are exemplary and explanatory only and are not restrictive of the invention, as claimed. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." The term "distal" refers to a portion farthest away from a user when introducing a device into a patient. By contrast, the term "proximal" refers to a portion closest to the user when placing the device into the patient. Proximal and distal directions are labeled with arrows marked "P" and "D", respectively, throughout the figures. Although endoscopes are referenced herein, reference to endoscopes or endoscopy should not be construed as limiting the possible applications of the disclosed aspects. For example, the disclosed aspects may be used with duodenoscopes, bronchoscopes, ureteroscopes, colonoscopes, catheters, diagnostic or therapeutic tools or devices, or other types of medical devices. Further, relative terms such as, for example, "about," "substantially," "approximately," etc., are used to indicate a possible variation of ±10% in a stated numeric value or range.

### BRIEF DESCRIPTION OF THE DRAWINGS

The accompanying drawings, which are incorporated in and constitute a part of this specification, illustrate exemplary aspects of the present disclosure and together with the description, serve to explain the principles of the disclosure.
FIG. 1 is a perspective view of an endoscope system and a magnified view of the distal end of the endoscope of the endoscope system, according to aspects of this disclosure.
FIGs. 2A-2D are perspective and front views of a device tip, according to aspects of this disclosure.
FIGs. 3A-3B are perspective and front views of a component of a device tip, according to aspects of this disclosure.
FIGs. 4A and 4B are perspective views of a system to rotate the component of FIGs. 3A-3B.
FIGs. 5A and 5B are perspective and front views of a device tip, according to aspects of this disclosure.
FIGs. 6A and 6B are perspective and front views of a device tip, according to aspects of this disclosure.
FIGs. 7A-7C are side views of internal components of a device tip, according to aspects of this disclosure.
FIG. 8 is a front view of some of the components of the device tip of FIGs. 7A-7C, according to aspects of this disclosure.
FIG. 9 is a perspective view of a portion of a device tip, according to aspects of this disclosure.
FIG. 10 is a perspective view of components of the device tip shown in FIG. 9, according to aspects of this disclosure.
FIGs. 11A-11D are front views of components of a device tip, according to aspects of the present disclosure.
FIG. 12 is a perspective view of a component of a device tip, according to aspects of the present disclosure.
FIGs. 13A-13C and 14 are perspective views of a working channel of a device tip including the component of FIG. 12, according to aspects of the present disclosure.
FIGs. 15A and 15B are front views of a device tip, according to aspects of the present disclosure.
FIGs. 16A and 16B are perspective views of a device tip, according to aspects of the present disclosure.

### DETAILED DESCRIPTION

Reference will now be made in detail to aspects of the present disclosure, examples of which are illustrated in the accompanying drawings. Wherever possible, the same or similar reference numbers will be used through the drawings to refer to the same or like parts.

Embodiments of this disclosure seek to improve stability for accessory devices (e.g., working tools) within a lumen or working channel of a scope, such as, e.g., an endoscope, for tools that are undersized relative to the lumen. A steerable component may be included in some embodiments, to give a user an additional degree of freedom when manipulating the tools at a distal end of the scope. Embodiments of this disclosure seek to improve a physician's ability to manipulate accessory devices within the working channel of the endoscope.

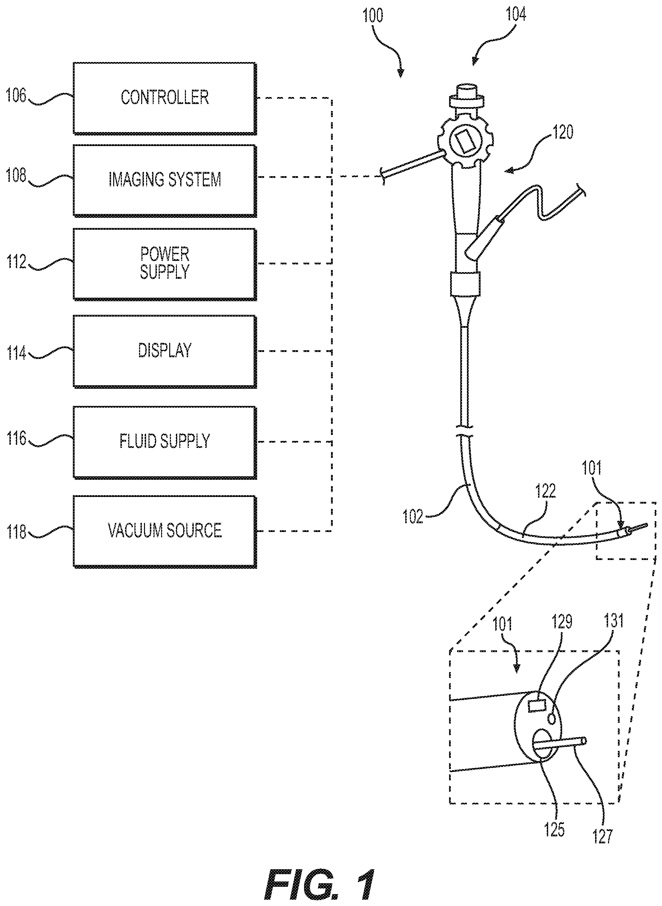

An exemplary endoscopy system 100 is shown in FIG. 1. Endoscopy system 100 may include an endoscope 104. Endoscope 104 may include a handle assembly 120 and a flexible tubular shaft 102. The flexibility of shaft 102 may be sufficient to allow shaft 102 to bend, to facilitate navigation of shaft 102 through a subject's tortuous anatomical passages. Shaft 102 may terminate at a distal tip 101. Shaft 102 may include an articulation section 122 for deflecting distal tip 101 in up, down, left, and/or right directions. In one example, articulation section 122 may provide for full retroflexion (e.g., rotation of distal tip 101 through an arc of 180 degrees) or only partial retroflexion (e.g., rotation of distal tip 101 through an arc of less than 180 degrees). Endoscope 104 also may include one or more lumens extending therethrough, and one or more openings in communication with the one or more lumens. For example, the one or more lumens may extend through handle assembly 120 and shaft 102, and the one or more openings may be on handle assembly 120 and distal tip 101. Endoscope 104 may be any suitable member for insertion into a patient's body, such as, e.g., an endoscope, a gastroscope, a ureteroscope, a nephroscope, a colonoscope, a hysteroscope, a ureteroscope, a bronchoscope, a cystoscope, a duodenoscope, a sheath, or a catheter.

One or more auxiliary devices may be operatively coupled to endoscope 104. Exemplary auxiliary devices may include a controller 106, an imaging system 108, a power supply 112, a display 114, a fluid supply 116, and/or a vacuum source 118, each of which is briefly described below. Controller 106 may include, for example, any electronic device capable of receiving, storing, processing, generating, and/or transmitting data according to instructions given by one or more programs. Controller 106 may be operatively coupled to, or part of, one or more of endoscope 104 and the other auxiliary devices, to control one or more aspects of their operation. Power supply 112 may include any suitable power source, and associated connectors (e.g., electrically-conductive wires), for supplying electronic components in the auxiliary devices and endoscope 104 with electrical power. Fluid supply assembly 116 may include a reservoir, a medical irrigation bag, a pump, and any suitable connectors (e.g., tubing for fluidly coupling fluid supply 116 and endoscope 104). The pump may supply a flow of pressurized fluid to one or more of the lumens in endoscope 104, and the pressurized fluid flow may be emitted from distal tip 101 and/or used to inflate expandable components present at distal tip 101. Vacuum source 118 may provide suction or vacuum pressure to one or more lumens of the endoscope, and thereby provide a suction force to draw material toward and/or into endoscope 104, and/or to deflate expandable components.

Imaging system 108 may include imaging electronics to, for example, process signals received from an image sensor in endoscope 104, send signals for controlling the image sensor, adjust illumination levels of areas being viewed by the image sensor, and/or facilitate the display of image sensor data on display 114.

Distal tip 101 may include one or more image sensors 129 and one or more illuminators 131, shown in the magnified view of distal tip 101 in FIG. 1. One or more image sensors 129 may include a charge-coupled device image sensor, a complementary metal-oxide image semiconductor, or the like coupled to a cable or wire running through the shaft 102 of endoscope 104. One or more illuminators 131 may include light emitting diodes (LEDs) or the like.

A tool 127 may be inserted into a lumen or working channel 125 of endoscope 104 and may exit out of the distal end of lumen 125. Tool 127 may include, for example, a guidewire, cutting or grasping forceps, a biopsy device, a snare loop, an injection needle, a cutting blade, scissors, a retractable basket, a retrieval device, an ablation and/or electrophysiology catheter, a stent placement device, a surgical stapling device, a balloon catheter, a laser-emitting device, and/or any other suitable therapeutic or diagnostic instrument. As shown in the magnified view of distal tip 101, tool 127 has a smaller circumference about its longitudinal axis compared to the circumference about the longitudinal axis of lumen 125, and may include a smaller cross-sectional diameter as compared to the diameter of lumen 125. Aspects of this disclosure provide embodiments of medical device tips, such as distal tip 101, that may facilitate fixedly coupling a tool, such as tool 127, to distal tip 101 so that when a user moves distal tip 101, tool 127 will also move in the same direction.

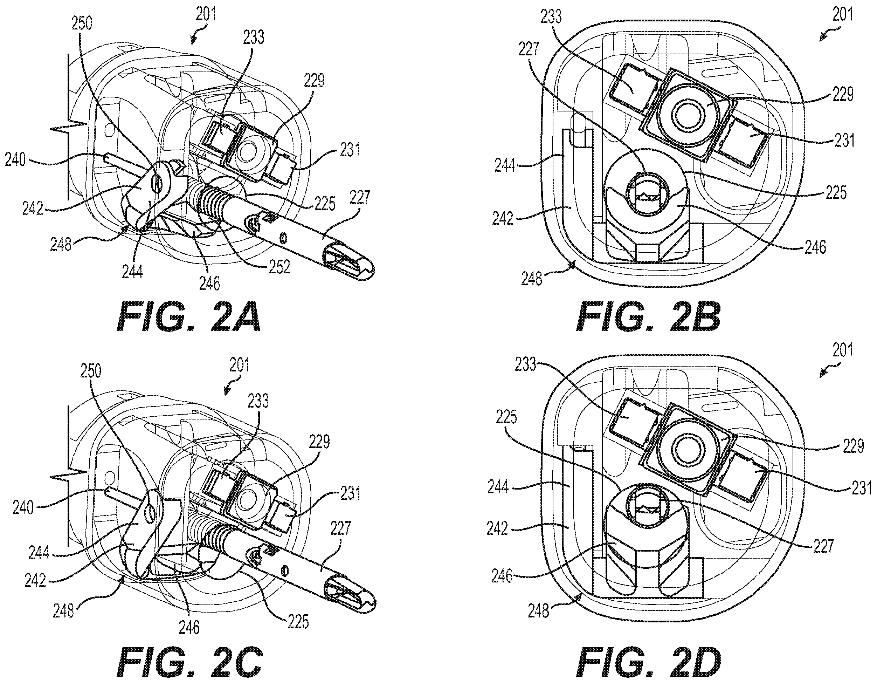

FIGS. 2A-2D show perspective and front views of a medical device tip 201 including an image sensor 229, illuminators 231, 233, a lumen or working channel 225, and an elevator 242. Tool 227 is shown positioned within working channel 225. Elevator 242 may provide a means for securing tool 227 to distal tip 201 by sandwiching tool 227 between elevator 242 and a radially-inner wall of working channel 225. Elevator 242 may also move tool 227 across the path of motion of elevator 242, for example moving tool 227 in sweeping motions across the path of motion of elevator 242. The path of tool 227 when engaging elevator 242 may be dependent on the starting position of tool 227 without elevator 242 engaged with tool 227. In some examples, the starting position of tool 227 may be affected by the stiffness of tool 227, articulation of distal tip 201, and/or the path of the medical device's movement through a patient's anatomy. In some examples, elevator 242 may be positioned at an angle relative to the longitudinal axis of the medical device tip 201 and may be configured to adjust the position of a tool positioned within a working channel that exits medical device tip 201 at a side wall, with a working channel distal opening positioned proximal to the distal front face of medical device tip 201 (note this configuration is not shown in the figures).

Elevator 242 may include a U-shaped surface, as will be explained in connection with a second extension 246, that accepts tool 227. Elevator 242 may be anchored to a portion of distal tip 201 at a proximal portion 248 of elevator 242. The proximal portion 248 of elevator 242 may be rotatable about an axis substantially transverse to the longitudinal axis of working channel 225. In some examples, proximal portion 248 may be rotatably coupled to a portion of distal tip 201 forming a hinge and may allow elevator 242 to rotate about an axis extending through proximal portion 248.

Elevator 242 may include a first extension 244 and a second extension 246, both of which may extend from proximal portion 248. In some examples, first extension 244 may be offset from second extension 246 (to the side of extension 246) and may be angled relative to second extension 246. In some examples, first extension 244 may be offset from the longitudinal axis of working channel 225 such that first extension is outside of working channel 225. In some examples, second extension 246 is positioned adjacent to or within working channel 225. An exterior surface 252 of second extension 246 is configured to substantially align with, or otherwise be flush with, a radially-inner surface of working channel 225. The radially-inner surface of working channel 225 may include a recessed portion in which second extension 246 of elevator is positioned. In some examples, a distal portion of first extension 244 may include a fastening chamber 250. Fastening chamber 250 may be configured to receive a cable or actuator 240. In some examples, a distal end of cable 240 may be rotatably coupled to a distal portion of first extension 244 such that when cable 240 is pulled in the proximal direction, first extension 244 is pulled proximally and pivots about an axis through proximal portion 248, and a portion of cable 240 may rotate about an axis extending through fastening chamber 250. Cable 240 may be stiff and/or non-compressible such that cable can be translated distally to move first extension 244 distally and cause elevator to rotate about an axis through proximal portion 248. In some examples, cable 240 may be coupled to a lever (not shown) positioned at handle 120 or another proximal portion of endoscope 104. In some examples, elevator 242 may be positioned less than 20mm from the distal front face.

In operation, elevator 242 may be configured to transition between a first configuration shown in FIGs. 2A and 2B to a second configuration shown in FIGs. 2C and 2D. A user may pull on a lever at a proximal portion of endoscope 104, which may pull cable 240 proximally. When cable 240 is pulled proximally, elevator 242 may rotate about an axis extending through proximal portion 248 and may transition from a first configuration shown in FIGs. 2A and 2B to a second configuration shown in FIGs. 2C and 2D. In some examples, the lever may be in an open position when the elevator 242 is positioned such that working channel 225 is open to allow tool 227 to move within working channel 225.

When a user moves the lever from an open position to a closed position, cable 240 may be moved proximally and elevator 242 (and specifically second extension 246) may eclipse a portion of working channel 225 (shown in FIGs. 2C and 2D). When elevator 242 eclipses a portion of working channel 225, section extension 246 extends within working channel 225 and may contact tool 227 so as to push tool 227 against a radially-inner surface of working channel 225. In some examples, tool 227 may remain parallel to the longitudinal axis of medical device tip 201 and/or working channel 225 when pushed against a radially-inner surface of working channel 225, and in other examples tool 227 may be tilted at an angle relative to the longitudinal axis of medical device tip 201 and/or working channel 225 when pushed against a radially-inner surface of working channel 225. A user may move the lever to a closed position and/or pull cable 240 proximally to rotate elevator 242 and push second extension 246 against tool 227 to hold 227 in position. The user may lock the lever in a closed position allowing the user to move distal tip 201 and tool 227 simultaneously and in concert, and without holding tool 227 separately. By pushing second extension 246 against tool 227, a user may stabilize tool 227 and prevent tool 227 from moving within working channel 225. The position of elevator 242 may be optimized so as to position tool 227 at any portion of the radially-inner surface of working channel 225 when elevator 242 engages tool 227, such as to position tool 227 at a specific location relative to image sensor 229 and/or illuminators 231, 233. In some examples, a lever controlling cable 240 may be configured to lock in a closed position to allow the user to lock elevator 242 at a position holding tool 227 in place. When the lever controlling cable 240 is configured to lock in a closed position, the user may not have to hold a proximal portion of tool 227 at a biopsy port of handle 120 and thus may potentially reduce user fatigue and allow the user to have a free hand.

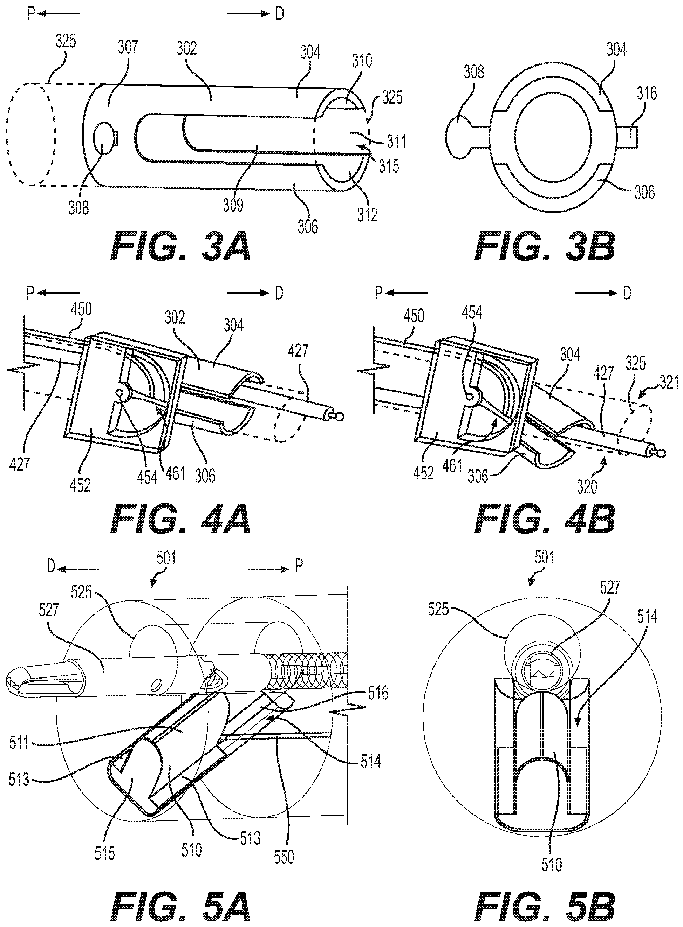

FIGs. 3A and 3B show an alternative embodiment of an elevator 302 that may be incorporated into a device tip. Elevator 302 may have any of the features previously described in relation to elevator 242. In FIG. 3A, elevator 302 is shown positioned relative to a working channel 325 that could be within endoscope 104 or a similar medical device. Elevator 302 includes a first extension 304, a second extension 306, and a proximal portion 307 connecting the first extension 304 to the second extension 306. First extension 304 may oppose second extension 306 and a lumen 315 may extend longitudinally the length of elevator 302. When elevator 302 is positioned within a distal tip of a device, such as distal tip 101, lumen 315 may longitudinally align with a working channel of the device. Radially inner surfaces 310, 312 of the first extension 304 and the second extension 306, respectively, may be curved and may be configured to conform to working channel 325. Proximal portion 307 may include a connecting portion 308. Connecting portion 308 may extend radially outward from the longitudinal axis of elevator 302 and may protrude from a radially-outer surface of elevator 302. Connecting portion 308 may be configured to connect to a mechanism that allows a user to rotate elevator 302 about connection portion 308, e.g. a mechanism similar to first extension 244 shown in FIGs. 2A-2D. Connecting portion 308 may be positioned outside or partially outside a working channel 325 and within a distal tip portion of a medical device. Tab 316 may be positioned on a radially-outer surface of elevator 302 at a portion of elevator 302 opposite connecting portion 308. Tab 316 may be rotatably coupled to a portion of a distal tip of a medical device, and may be positioned partially outside of working channel 325. In some examples, first extension 304 and second extension 306 may be longitudinal, curved arms extending from an annular portion 307. Annular portion 307 may have a proximal opening to receive an endoscopic tool. First extension 304 and second extension 306 may define two longitudinal slots 309 therebetween. Distal opening 311 may be at a distalmost end of elevator 302 and may be configured for tool 325 to extend through.

In some examples, connection portion 308 may be coupled to a rotation body 452. Rotation body 452 may including a rotatable hub 454 which is coupled to connecting portion 308. A cable or actuator 450 positioned outside of working channel 325 may be fixedly coupled to rotatable hub 454, longitudinally offset from hub 454. Cable 450 may extend through a lumen in rotation body 452 and may be coupled to a extension 461 that extends from rotatable hub 454. When a user moves cable 450 distally, extension 461 may rotate as a result from the force applied by cable 450 to extension 461, and rotatable hub 454 may rotate via the movement of extension 461, thus rotating elevator 302 through connecting portion 308, and deflecting elevator 302. In some examples, deflecting elevator 302 may move first extension 304 such that first extension 304 eclipses working channel 325 and rotates towards a first side 320 of working channel 325. When first extension 304 eclipses working channel 325, first extension 304 may contact tool 325 and push tool 427 towards first side 320 of working channel 325 to hold tool 427 between first side 32.0 and first extension 304, and thus prevent movement of tool 427 within working channel 325. When a user moves cable 450 proximally, cable 450 may pull on extension 461 and rotatable hub 454 may rotate via the movement of extension461, thus rotating elevator 302 through connecting portion 308, and deflecting elevator 302 such that second extension 306 eclipses working channel 325 and rotates towards a second side 321 of working channel 325. In some examples, as first extension 304 or second extension 306 occludes a portion of working channel 325, tool 427 may move across working channel 325.

Cable 450 may be moved by the user in the same manner as described hereinabove in relation to cable 240. In some examples, a user may push or pull cable 450 which will result in rotation of extension 461 about rotation hub 454, and rotation of extension 461 deflects either first extension 304 or second extensions 306 over a portion of working channel 325. Since elevator 302 allows a user to position first extension 304 within working channel 325 to hold tool 427 toward first side 320 of working channel 325, and to position second extension 306 within working channel 325 to hold tool 427 toward second side 321 of working channel 325, elevator 302 provides a user with the ability to hold tool 427 at multiple different locations within working channel 325. In some examples, elevator 302 may be positioned partially within working channel 325 when the longitudinal axis of elevator 302 is parallel to the longitudinal axis of working channel 325, and in other examples elevator 302 may be positioned entirely outside of working channel 325 when the longitudinal axis of working channel 325 is parallel to the longitudinal axis of elevator 302. In some examples, the radially-inner surfaces 310, 312 of elevator 302 may be aligned with the radially inner surfaces of working channel 325.

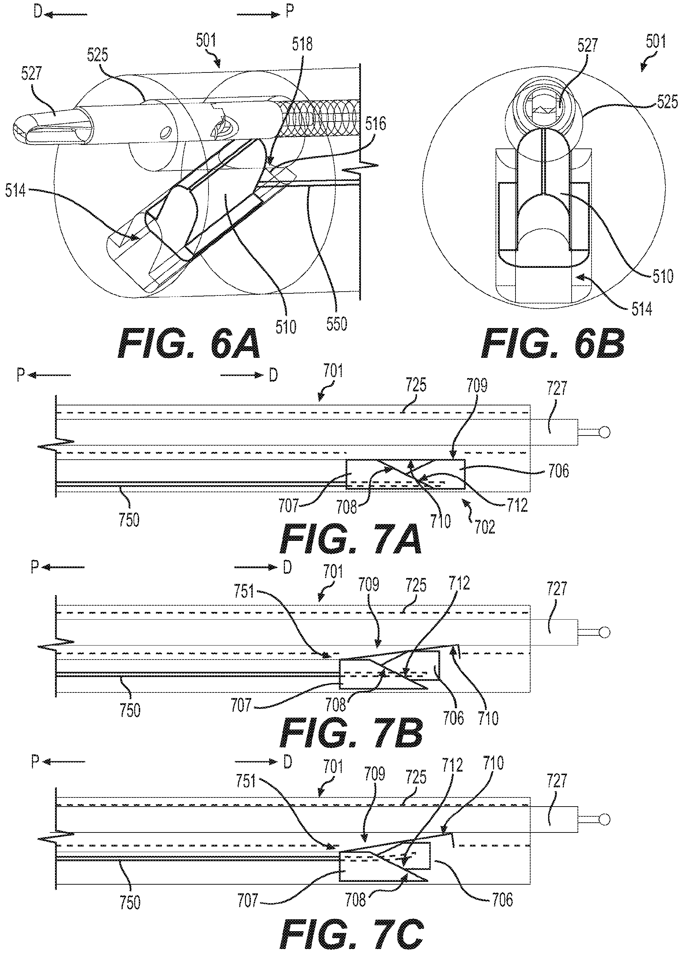

FIGs. 5A-5B and 6A-6B show perspective and front views of another elevator 510 in a device tip 501. In some examples, elevator 510 may include a U-shaped convex exterior surface 511 with opposing flanges 513 extending radially outward from a bottom portion of the U-shaped exterior surface 511. In other examples, the exterior surface of elevator 510 may be any suitable shape configured to extend through opening 518 to contact tool 527. Flanges 513 define upper surfaces of a bottom portion 515 of elevator 510. Bottom portion 515 may be positioned within, and slide within, a channel 514 that extends from an opening 518 in the radially-inner surface of working channel 525. Elevator 510 and/or channel 514 may have a longitudinal axis substantially transverse to working channel 525. In some examples, channel 514 may extend at an angle distally from opening 518 in working channel 525. In other examples (not shown), channel 514 may extend proximally from opening 518 at an angle relative to the longitudinal axis of working channel 525. Elevator 510 may be configured to move within channel 514. In some examples, channel 514 may include an opening 516 extending longitudinally within channel 514. Opening 516 may be configured to receive cable or actuator 550 and may allow cable or actuator 550 to move within opening 516. In some examples, elevator 510 may be within an endoscope cap that is attachable to a distal tip of an endoscope.

Cable or actuator 550 may be fixedly coupled to elevator 510 or may be rotatably coupled to elevator 510 such that cable may rotate about the point at which cable 550 is coupled to elevator 510. In some examples, cable 550 may extend from elevator 510 to a proximal portion of the device such that a user may move cable 550 when distal tip 501 is positioned within a body of a patient. Cable 550 may be stiff and/or non-compressible such that cable 550 can be translated distally or proximally to move elevator 510 distally or proximally, and cause elevator to move within channel 514. In some examples, cable 550 may be coupled to a lever (not shown) positioned at handle 120 or another proximal portion of endoscope 104, in a similar manner as described above in relation to elevator 242.

FIGs. 5A and 5B show elevator 510 completely received within channel 514 such that elevator 510 does not extend within working channel 525. When a user translates cable 550 proximally, such as by actuating a lever on handle 120 to pull cable 550 proximally, elevator 510 may be translated within channel 514 and extend within working channel 525, eclipsing working channel 525. In other examples (not shown), translating cable 550 distally will move elevator 510 into working channel 525. In some examples, translating cable 550 proximally transitions elevator 510 from a first configuration shown in FIGs. 5A and 5B to a second configuration shown in FIGs. 6A and 6B. When a tool 527 is positioned within working channel 525, moving elevator 510 into working channel 525 may move tool 527. By pulling cable 550 proximally, a user may move elevator 510 within working channel 525 and sandwich tool 527 between elevator 510 and the radially-inner surface of working channel 525 (shown in FIGs. 6A and 6B). When a user moves elevator 510 such that elevator 510 pushes on tool 527 and holds tool 527 between elevator 510 and a radially-inner surface of working channel 525, tool 527 may be held in place and the user may move distal tip 501 and tool 527 in unison without tool 527 moving within working channel 525. By holding a tool 527 at a particular position within working channel 525, elevator 510 may help stabilize tool 527 within working channel 525. In other examples, the angle, shape, and/or size of elevator 510 may be optimized to achieve preferred elevator performance and accessory device compatibility. In some examples, elevator 510 may be moved from a position within working channel 525 to a position within channel 514 outside working channel 525 by moving a tool 527 through working channel 525.

FIGs. 7A-7C show another embodiment of an elevator system 702 including a wedge 706 positioned within a distal tip 701 of a medical device. FIGs. 7A-7C show side views of distal tip 701 within working channel 725, a tool 727 positioned within working channel 725, wedge 706, and an opening 709 in the radially-inner surface of working channel 725. Opening 709 connects a cavity (not shown), in which wedge 706 is positioned, with working channel 725. Wedge 706 may include a pair of surfaces substantially transverse to the longitudinal axis of working channel 725 that meet at an edge positioned at a proximal end of wedge 706, and this pair of surfaces may form a wedge. In some examples, wedge 706 may be anchored to a positioned outside of working channel 725 by a biasing member, such as a spring, coupled to wedge 706 and to a portion of distal tip 701. The biasing member (not shown) may be biased to hold wedge 706 at a position outside of working channel 725 with elevator longitudinal axis substantially parallel to working channel 725. In some examples, wedge 706 may be biased away from working channel 725 by tab member 710. At least one angled surface 712 of wedge 706 may be configured to align with an angled surface 708 of block 707. In some examples, a biasing member, such as a coil spring, may be coupled to hinge 751 and the coil spring may bias hinge 751 towards block 707 and/or wedge 706, and pulling cable or actuator 750 proximally may slide wedge 706 towards tab member 710 and push tab member 710 towards working channel 725 against the force of the biasing member.

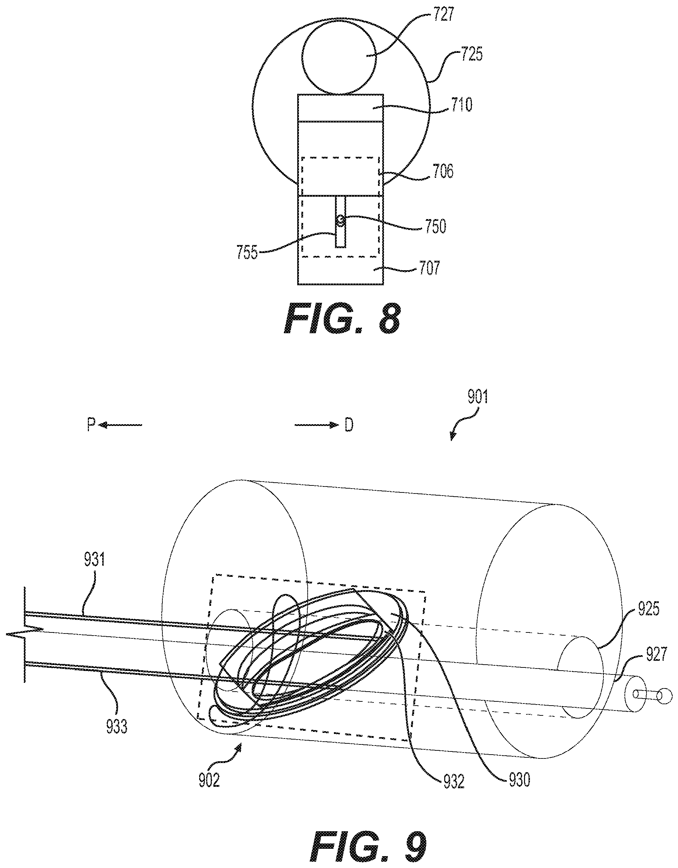

Block 707 may be fixedly coupled to distal tip 701 such that block 707 does not move relative to wedge 706. In some examples, when wedge 706 and block 707 do not move relative to each other, position of the tab member 710 does not change. Block 707 may have a longitudinal axis substantially parallel to working channel 725 and an angled surface 708 substantially transverse to the longitudinal axis of working channel 725. Surface 708 may be configured to align with a surface of wedge 706. Block 707 may include a channel 755 (shown in FIG. 8) extending longitudinally through block 707. FIG. 8 shows block 707 including channel 755 and cable 750, and positioning of block 707 relative to wedge 706 and working channel 725. Tab member 710 is not shown in FIG. 8. Channel 755 may be configured to receive cable 750 and may allow cable 750 to translate proximally and distally through channel 755. Channel 755 may also prevent cable 750 from extending outside the radially-outermost portion of block 707 so as to limit the distance wedge 706 may move towards working channel 725. Cable 750 may be fixedly coupled to wedge 706 (shown in dotted lines in FIG. 8) such that moving cable proximally moves wedge 706 proximally, and moving cable 750 distally moves wedge 706 distally.

A tab member 710 may extend from a proximal portion of distal tip 701 to a distal portion. Tab member 710 may be rigid and may include a hinge 751 at a proximal portion of tab member 710. In other examples, tab member 710 may be flexible, and hinge 751 may be a living hinge. In some examples, hinge 751 may include a coil spring or other biasing member. The distalmost end of tab member 710 may be positioned adjacent to opening 709 such that when tab member 710 pivots about hinge 751, tab member 710 may extend within working channel 725. In some examples, tab member 710 may be abutting an exterior surface of block 707 and an exterior surface of wedge 706. Tab member 710 may be configured to contact tool 727 and hold tool 727 at a position within working channel 725. Tab member 710 may be biased away from working channel 725, due to hinge/coil spring 751, such that tab member 710 moves to a position outside of working channel 725 when there is no force applied to tab member 710. Wedge 706, tab member 710, and block 707 may be made of any suitable, biocompatible material and may be sufficiently rigid to operate as described herein.

In operation, a user may move cable 750 proximally, thus pulling wedge 706 proximally. Angled surface 712 of wedge 706 may slide across angled surface 708 of block 707 as wedge 706 moves proximally, thus moving wedge 706 towards working channel 725. As wedge 706 moves towards working channel 725, wedge 706 moves tab member 710 towards working channel 725. As tab member 710 moves towards working channel 725, tab member 710 also rotates about hinge 751. When tab member 710 moves towards working channel 725, tab member 710 extends through opening 709 into working channel 725 and may contact tool 727. Thus, when a user pulls cable 750 proximally, tab member 710 will move into working channel 725 and push tool 727 towards a radially-inner surface of working channel 725. As shown in FIG. 7C, after a user pulls cable 750 proximally, tab member 710 may hold tool 727 against a radially-inner surface of working channel 725 and prevent movement of tool 727 within working channel 725. Tool 727 may be deflected by tab member 710 extending within working channel 725. In some examples, a lever controlling cable 750 may be configured to lock in a closed position to allow the user to lock wedge 706 at a position in which tab member 710 is holding tool 727 against a radially-inner surface of working channel 725, in a similar manner as discussed previously in relation to elevators 242, 302, and 510. To release tool 727 and allow tool 727 to move within working channel 725, a user may move cable 750 distally, and thus move wedge 706 distally. Alternatively, releasing cable 750 from proximal position will allow tab member 710 to pivot about hinge 751 to automatically move wedge 706 distally.

While FIGs. 7A-7C show wedge 706 positioned within distal tip 701, an alternative embodiment (not shown) may include wedge 706, block 707, and distal portion 709 positioned distal to distal tip 701 and/or outside the body 760 of distal tip 701.

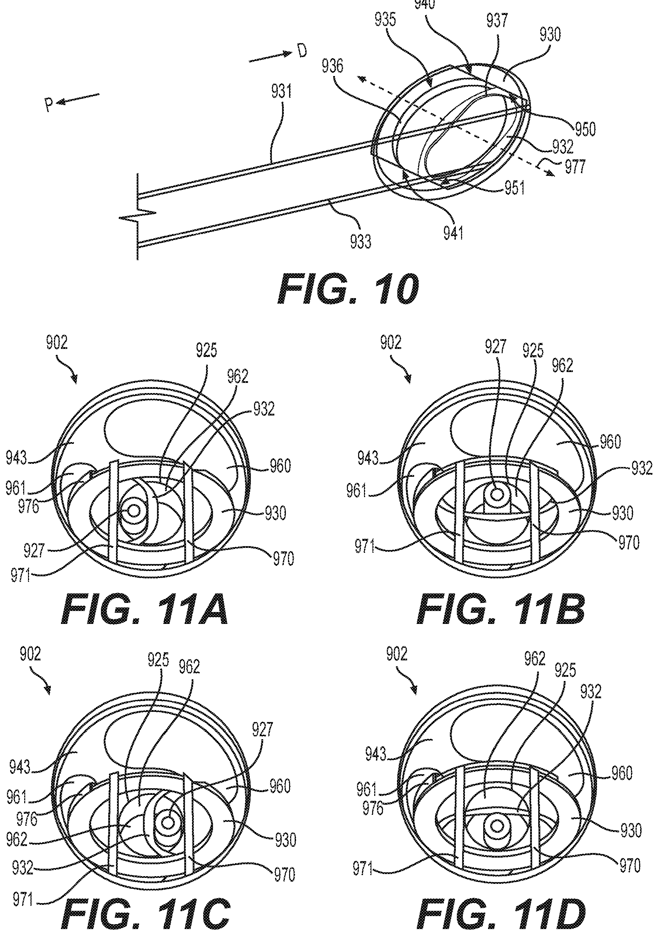

FIGs. 9-11D show another embodiment of an elevator assembly 902 including a rotating plate 930, a slide 932, and a frame 943 (shown in FIGs. 11A-11D) that may be positioned within a distal tip 901 of a medical device. Plate 930 may include a circular opening or lumen 936 extending through a central portion of plate 930 and a recess 935 extending from a first edge 941 to an opposing second edge 940 of plate 930. In some examples, plate 930 may have a circular outer shape. A cable or actuator 931 may be fixedly coupled to plate 930. Cable or actuator 931 may be configured to move plate 930, for example rotate plate 930 within a recessed portion of frame 940. Recess 935 may be configured to receive slide 932 and may include opposing straight edges 941, 940 configured to slidably engage slide 932. The circumference of lumen 936 may be configured to be larger than the circumference of working channel 925 to allow working channel 925 to extend through lumen 936.

Slide 932 may have a generally circular outer shape and may include an opening or lumen 937 extending through a central portion of slide 932. Slide 932 may include opposing straight edges 950, 951. Opposing straight edges 950, 951 may be configured to align with opposing straight edges 940, 941 of recess 935 such that edges 950, 951 slidable interface with edges 940, 941. Slide 932 may be configured to translate within recess 935 and be limited by recess 935 to moving along a longitudinal axis 977 of recess 935. In some examples, the interface between edges 940, 941 and edges 950, 951 may prevent rotation of slide 932 relative to plate 930 when a force is applied to slide 932 via cable 933. As shown in FIG. 9, lumen 937 may be sized to allow working channel 925 to extend through lumen 937. Slide 932 may be fixedly coupled to cable 933. In some examples, cable 933 may be rigid and may be configured to move slide 932 within recess 935. Translation of cable 933 either proximally or distally may move slide 932 along longitudinal axis 977.

FIGs. 11A-11D show elevator assembly 902 including plate 930, slide 932, and frame 943. Elevator assembly 902 is shown in FIGs. 11A-11D positioned relative to a tool 927 without showing other components of a distal tip of a medical device. In some examples, frame 943 may have a circular outer shape and may include a plurality of lumens 960, 961, 962 to allow components of a medical device, including components of elevator assembly 902 to extend through and move within the plurality of lumens 960, 961, 962. For example, frame 943 may include one or more lumens 960, 961 configured to allow cables from one or more image sensors and/or one or more illuminators to extend through. In some examples, frame 943 may be part of the distal tip 901 of a medical device and formed within a portion of the distal tip 901. Lumen 962 may be configured to align with a working channel, such as working channel 925 of device tip 901. Frame 943 may be fixedly coupled to and/or incorporated within a distal tip 901 of a medical device. Frame 940 may be configured to hold rotating plate 930 and slide 932 such that rotating plate 930 may rotate relative to frame 940, and slide 932 may translate within recess 935. Frame 940 may include one or more brackets 970, 971 configured to couple plate 930 and slide 932 to frame 943, while allowing plate 930 and slide 932 to move relative to frame. A recess portion 976 may prevent slide 932 from moving beyond the radially-outermost portion of plate 930. In some examples, frame 943 may limit the amount of rotation of plate 930 to ninety degrees of rotation, such as by having a lumen 961 configured to allow cable 931 to rotate plate 930 ninety degrees and to stop rotation of plate 930 via edges of lumen 961 contacting cable 931. Frame 943 may be positioned within a distal tip, such as distal tip 901, at an angle relative to the longitudinal axis of the distal tip 901. By positioning frame 943 at an angle relative to the longitudinal axis of distal tip 901, a user may translate cable 931 proximally or distally to cause plate 930 to rotate relative to frame 943 since plate 930 may rotate within frame 943 but not translate proximally or distally relative to frame 943. Also, positioning frame 943 at an angle relative to the longitudinal axis of distal tip 901 may facilitate movement of slide 932 via push/pull of cable 933. In some examples, rotation of cable 931 may rotate plate 930. In some examples, protrusions or other "hard stop" features may be incorporated into rotating plate 930 that may interact with features on frame 943, such as protrusions on frame 943, to limit rotation of plate 930 beyond a desired range.

In operating a medical device with a distal tip including elevator assembly 902, a user may first rotate rotating plate 930 by moving cable 931 in a proximal or distal direction, which may cause the distal end of cable 931 to move in a direction substantially transverse to the longitudinal axis of distal tip 901 or otherwise about the longitudinal axis of tip 901. When a user rotates plate 930, an axis of movement 977 of slide 932, which corresponds to the longitudinal axis of recess 935, rotates, and slide 932 moves to a different position relative to working channel 925. After rotating plate 930, a user may move slide 932 through recess 935 by moving cable or actuator 933. By moving slide 932, a portion of slide 932 may move within working channel 925 and may contact tool 927. A user may move slide 932 such that slide 932 contacts tool 927 and pushes tool 927 against a radially-inner surface of working channel 925, which may hold tool 927 against a radially inner surface of working channel 925. FIGs. 11A-11D show various positions in which tool 927 may be held against a radially-inner wall of a working channel, such as working channel 925, using elevator assembly 902. As shown in FIGs. 11A-11D, a user may rotate plate 943 and translate slide 932 to position tool 927 against any point along the radially-inner surface of working channel 925. In some examples, elevator assembly 902 may allow a user to move a tool 927 to a desired position within a working channel after tool 927 has been stabilized within working channel 925, such as by rotating plate 930 after tool 927 has been pinned against a side of working channel 925 via slide 932. Elevator assembly 902 may allow the use of larger working channels and/or smaller tools, because elevator assembly 902 provides a means for a user to stabilize a tool within a working channel at a user defined location, and may allow for more predictability in tool orientation.

In some examples (not shown), plate 930 may be rotated via a mechanism in which the user rotates a knob of the medical device to actuate rotation of plate 930, and slide 932 may be translated via a mechanism in which the user rotates a knob of the medical device to move slide 932. For example, rotation of an actuator around a pivot point within a handle of a medical device, such as handle 120, may push (move distally) or pull (move proximally) a cable (such as cable 931 or cable 933). In some examples where movement of plate 930 or slide 932 is controlled by translating a cable, a user may rotate a lever about a pivot point, which may extend or retract an arm within a handle of a medical device, such as handle 120, to subsequently push or pull the cable. In other examples, a proximal end of a cable (such as cable 931 or cable 933) may be coupled to a dial, and the dial may be positioned such that rotation of the dial rotates the cable about the cable's longitudinal axis.

In other examples, an elevator assembly may include slide 932 and frame 943, without a recess to receive plate 930 and including a recess configured to receive slide 932 similar to recess 935. In this example, plate 930 may be fixed to a distal tip of a medical device in the same manner as elevator assembly 902 and slide 932 may be limited to a single axis of movement 977 since orientation of slide 932 and the recess in the frame is fixed. In this example of an elevator assembly, a single cable would be required to move slide 932 within the recess of the frame.

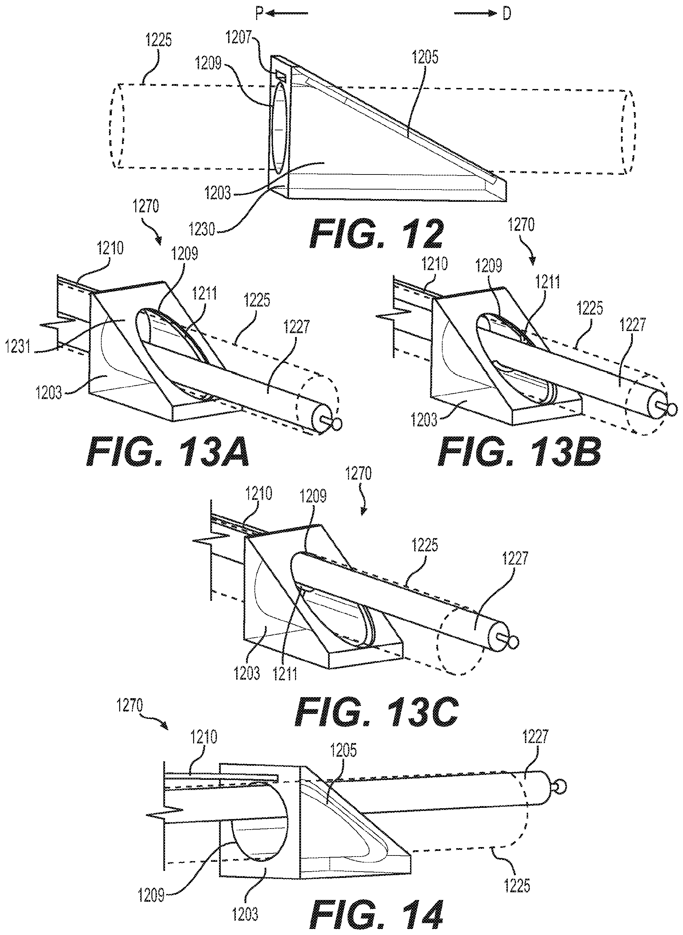

FIGs. 12-14 show another embodiment of an elevator assembly 1270. Elevator assembly 1270 may include a support block 1203, a snare loop 1211, and a cable or actuator 1210 coupled to the snare loop 1211. FIG. 12 shows support block 1203 without snare loop 1211 or cable 1210. Support block 1203 may include a lumen 1209 extending through a central portion of support block 1203 and along the longitudinal axis of support block 1203. Lumen 1209 may be configured to align with a working channel 1225 of a device tip (note working channel 1225 is shown in dotted lines for demonstrative purposes). Lumen 1209 may extend from an opening in a first proximal surface 1230 of block 1203 to an opening in a second distal surface 1231 of block 1203. First surface 1230 may be substantially perpendicular to the longitudinal axis of working channel 1225, and second surface 1231 may be substantially transverse to the longitudinal axis of working channel 1225. In some examples, second distal surface 1231 may be angled such that the distance between first surface 1230 and second surface 1231 increases as second surface 1231 extends distally. A channel 1205 may be positioned at the radially-irmer surface of lumen 1209 and proximate to second surface 1231.

In some examples, channel 1205 may be configured to receive snare loop 1211. Channel 1205 may extend circumferentially around the radially-inner surface of lumen 1209 and may be connected to, or otherwise in communication with, exit lumen 1207. Exit lumen 1207 may extend from channel 1205 to an opening at first surface 1230. Channel 1207 may be configured to receive snare loop 1211 and/or cable 1210. In some examples, when block 1203 is positioned such that working channel 1225 extends through lumen 1209, channel 1207 is positioned at a portion outside of working channel 1225. A gap in the radially-inner wall of working channel 1225 may be positioned adjacent to channel 1205 to allow snare loop 1211 to move into and out of working channel 1225 from channel 1205. Channel 1205 may be sized to allow snare loop 1211 to snap into channel 1205 and ride within channel 1205.

FIGs. 13A-13C show elevator assembly 1270 with snare loop 1211 received within channel 1205 (FIG. 13A), snare loop 1211 partially exited from channel 1205 and positioned within working channel 1225 (FIG. 13B), and snare loop 1211 holding tool 1227 against a radially inner surface of lumen 1209 (FIG. 13C). Snare loop 1211 may be coupled to cable 1210 within channel 1205, channel 1207, or outside of block 1203. Snare loop 1211 may be sufficiently stiff to allow a user to translate cable 1210 proximally to move snare loop 1211 from a position outside of channel 1205 to a position within channel 1205. A distal tip of a medical device may include a cavity within or adjacent to working channel 1225 that may be configured to receive block 1203 such that block 1203 may not move proximally or distally relative to the distal tip.

In some examples, cable 1210 may be coupled to a lever (not shown) positioned at a handle of a medical device, such as handle 120 or another proximal portion of endoscope 104. A user may pull on the lever at a proximal portion of endoscope 104, which may pull cable 1210 proximally. When cable 1210 is pulled proximally, snare loop 1211 may move out of channel 1205 and into working channel 1225. In some examples, the lever may be in an open position when the snare loop 1211 is positioned within channel 1205 and working channel 1225 is open to allow tool 1227 to move within working channel 1225. In some examples, the lever may be in a closed position when the snare loop 1211 is positioned within working channel 1225 and holding tool 1227 against a radially inner surface of lumen 1209, thus stabilizing tool 1227 within working channel 1225 and preventing tool 1227 from moving within working channel 1225.

In some examples, cable 1210 may include a sheath portion with a lumen extending therethrough, and a snare loop may include a proximal extension positioned within that lumen. In this example, a user may pull the proximal extension proximally to deploy snare loop 1211 within working channel 1225, and a portion of snare loop 1211 may be received by the sheath.

FIG. 14 shows an alternative perspective view of elevator assembly 1270 with snare loop 1211 positioned in the same position as in FIG. 13C.

In operating a medical device with a distal tip including elevator assembly 1270, a user may first position tool 1227 within working channel 1225. A user may then pull cable 1210 proximally in order to pull snare loop 1211 out of channel 1205 and position snare loop 1211 within working channel 1225. As a user pulls cable 1210 proximally, snare loop 1211 will contact tool 1227 since a proximal portion of snare loop 1211 will be pulled through channel 1207. Tool 1227 may be moved across working channel 1225 and then may be held against a radially inner surface of working channel 1225. By holding tool 1227 against a radially inner surface of working channel 1225, tool 1227 may be stabilized within a working channel 1225. When a user would like to release tool 1227 and create more space within working channel 1225, the user may move cable 1210 distally, thus moving snare loop within channel 1205 and out of working channel 1225. In some examples, when a user moves cable 1210 proximally snare loop 1211 slides back into channel 1225. In some examples, snare loop 1211 may retract, or snap back into place within channel 1225 due to its own rigidity paired with the geometry of block 1203. In some examples, snare loop 1211 may have a curvature that facilitates placement of snare loop 1211 within channel 1225. Block 1203 may include angled surfaces that facilitate movement of snare loop 1211 into channel 1225. In other examples, a biasing member, such as a spring, may be coupled to snare loop 1211 and may push/pull snare loop 1211 within channel 1225.

In some examples, working channel 1225 may include multiple elevator assemblies (blocks 1203, snare loops 1211, and cables 1210) positioned along working channel 1225 with each block 1203 rotated relative to each other block 1203. By using multiple blocks 1203 and snare loops 1211, a user may have the ability to move tool 1227 to different areas within working channel 1225 and stabilize tool 1227 at different positions.

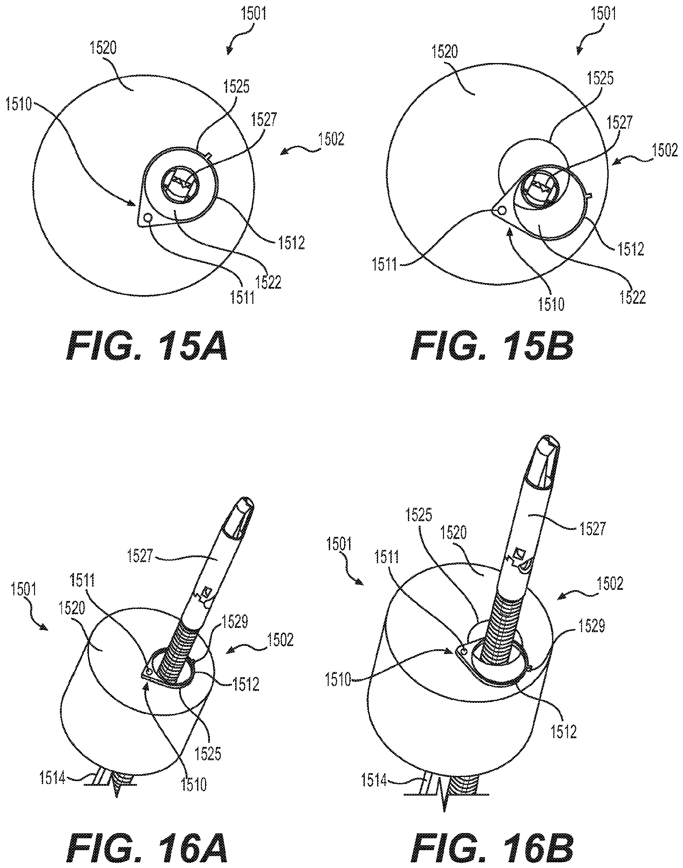

FIGs. 15A-16B show another embodiment of an elevator assembly 1502 positioned in a distal tip 1501 of a medical device. Elevator assembly 1502 may include an elevator 1512 and a cable or actuator 1514. Elevator 1512 may have a substantially circular outer shape and may be positioned at a distal front face of distal tip 1501. Elevator 1512 may include an opening or lumen 1522 extending through elevator 1512. In some examples, the longitudinal axis of lumen 1522 may be parallel to the longitudinal axis of distal tip 1501. Lumen 1522 may be configured to align with working channel 1525. In some examples, elevator 1512 may be positioned at the distal front face 1520 such that lumen 1522 is aligned with an opening of working channel 1525. Elevator 1512 may further include a cavity 1511 configured to receive and couple to cable 1514. In some examples, cavity 1511 may act as a pivot point when cable 1514 is coupled to elevator 1512 and positioned within cavity 1511, such that elevator 1512 may rotate about a pivot point positioned in cavity 1511 when a user rotates cable 1514 about an axis of cable 1514.

Cable 1514 may extend from elevator 1512 to a proximal portion of the medical device. Cable 1514 may be rigid such that rotation of a proximal portion of cable 1514 may result in rotation of a distal portion of cable 1514. Cable 1514 may extend through a lumen (not shown) in distal tip 1501. A distal portion of cable 1514 may be fixedly coupled to elevator 1512, such as coupled to and positioned within cavity 1511. A proximal portion of cable 1514 may be fixedly coupled to a knob, such as a knob positioned on handle 120 (see FIG. 1), and rotation of the knob may result in rotation of elevator 1512 at distal tip 1501. When elevator 1512 rotates, a portion of elevator 1512 may contact tool 1527 and may move tool 1527 towards a radially inner surface of working channel 1525. Accordingly, rotation of elevator 1512 may provide the user with means to move tool 1527 and hold tool 1527 against a radially inner surface of working channel 1525, thus preventing movement of tool 1527 within working channel 1525.

In some examples, elevator 1512, cable 1514, and/or a knob coupled to cable 1514 may be coupled to a biasing member, such as a spring, to bias elevator 1512 towards a position in which lumen 1255 is aligned with working channel 1525, as shown in FIGs. 15A and 16A. In some examples, a biasing member may be coupled to a protrusion 1529 of elevator 1512 (e.g. a spring attached to protrusion 1529 and distal face 1520 of tip 1501). A user may rotate cable 1514 such that elevator 1512 rotates and eclipses working channel 1525, and then when the user releases cable 1514 elevator 1512 will be moved by the biasing member back to a position in which lumen 1522 is aligned with working channel 1525.

In operating a medical device with a distal tip 1501 including elevator assembly 1502, a user may first position tool 1527 within working channel 1525. A user may then rotate cable 1514, via a knob or any other means, to rotate elevator 1512 and cause a portion of elevator 1512 to move towards a central longitudinal axis of working channel 1525 and cause a portion of elevator 1512 to extend across the distal opening of working channel 1525. A s a user rotates cable 1514, elevator 1512 will contact tool 1527 and move tool 1527 towards a radially inner surface of working channel 1525. Tool 1527 may be moved through working channel 1525 and then may be held against a radially inner surface of working channel 1525. By holding tool 1527 against a radially inner surface of working channel 1525, tool 1527 may be stabilized within working channel 1525. When a user would like to release tool 1527, the user may rotate cable 1514 in the opposite direction, thus moving elevator 1512 into alignment with a distal opening of working channel 1525 and moving a portion of elevator 1512 that was extending across the distal opening of working channel 1525 away from a central longitudinal axis of working channel 1525.

In some examples, cable or actuator 1514 may be prevented from rotating in a clockwise or counterclockwise direction when lumen 1522 is aligned with working channel 1525, via a stop component within distal tip 1501 (not shown) or a protrusion extending from distal front face 1520 (not shown). Preventing rotation of elevator 1512 in a counterclockwise or clockwise direction when lumen 1522 is aligned with working channel 1525 may facilitate a user positioning lumen 1522 back into alignment with working channel 1525 after stabilizing tool 1527 with elevator 1512.

In some examples, a distal tip of a medical device may include a plurality of elevators with the same structure as elevator 1512 but with pivot points located at different positions. Using multiple elevators 1512 may allow a user to stabilize tool 1527 and/or couple tool 1527 to distal tip 1501 at multiple different positions within working channel 1525.

Any of the disclosed embodiments of elevator assemblies may be positioned proximal to the distal tip of an endoscope or other medical device. For example, any of the disclosed embodiments of elevator assemblies may be positioned proximal to an articulation joint of an endoscope and/or proximal to an articulation section of an endoscope. Any of the disclosed embodiments of elevator assemblies may include an adjustable locking mechanism and/or adjustable actuator, such as a ratchet, to adjust an elevator's position and accommodate different size tools.

## Claims

1. A device (104), comprising:
a shaft (102) having a distal end (101, 201) and a lumen (225), the lumen (225) terminating in a distally-facing opening, wherein an instrument (227) is extendable through the lumen (225) and out of opening; and
an elevator (242) for engaging the instrument (227), the elevator (242) including:
an actuator (240) extending through at least a portion of the shaft (102); and
a body (244, 246, 248) coupled to the actuator (240), wherein a portion of the body (244, 246, 248) is configured to extend within the lumen (225) for selectively positioning the instrument (227),
wherein:
the body (244, 246, 248) includes a first extension (244), a second extension (246), and a proximal portion (248) connecting the first extension (244) and the second extension (246), the second extension (246) having a U-shaped body surface for engaging the instrument (227);
the actuator (240) is coupled to a distal portion of the first extension (244);
a portion of the second extension (246) is configured to move within the lumen (225) when the actuator (240) is moved proximally;
the portion of the second extension (246) is configured to move out of the lumen when the actuator (240) is moved distally; and
an exterior surface (252) of the second extension (246) is configured to align or be flush with a radially-inner surface of the lumen (225).

2. The device (104) of claim 1, wherein a longitudinal axis of the first extension (244,) is transverse to a longitudinal axis of the second extension (246).

3. The device (104) of any one of claims 1-2, wherein the body rotates about an axis positioned within the proximal portion (248) when the actuator (240) is moved proximally or distally.

4. A device comprising:
a shaft (102) having a distal end (101, 201) and a lumen (325), the lumen (325) terminating in a distally-facing opening, wherein an instrument (427) is extendable through the lumen (325) and out of opening; and
an elevator (302) for engaging the instrument (427), the elevator (302) including:
an actuator (450) extending through at least a portion of the shaft (102); and
a body (304, 306, 307, 452) coupled to the actuator (450), wherein a portion of the body (304, 306, 307, 452) is configured to extend within the lumen (325) for selectively positioning the instrument (427),
the body (304, 306, 307, 452) includes a first extension (304), a second extension (306), a proximal portion (307) connecting the first extension (304) and the second extension (306), and a pivot member (452), a radially inner surface of the first extension (304) and a radially inner surface of the second extension (306) being configured to align with a radially inner surface of the lumen (325);
the actuator (450) is coupled to the pivot member (452) and contacts a curved surface of pivot member (452);
a portion of the first extension (304) is configured to move within the lumen (325) when the actuator (450) is moved proximally; and
a portion of the second extension (306) is configured to move within the lumen when the actuator (450) is moved distally.

5. The device of claim 4, wherein the actuator (450) is coupled to the pivot member (452) at a position offset from the axis of rotation of the body (304, 306, 307, 452); and wherein a longitudinal axis of the first extension (304) and a longitudinal axis of the second extension (306) are parallel.

6. A device, comprising:
a shaft (102) having a distal end (501) and a lumen (525), the lumen (525) terminating in a distally-facing opening, wherein an instrument (527) is extendable through the lumen (525) and out of opening; and
an elevator (510) for engaging the instrument (527), the elevator (502) including:
an actuator (550) extending through at least a portion of the shaft (102); and
a body coupled to the actuator (550), wherein a portion of the body is configured to extend within the lumen (525) for selectively positioning the instrument (527),
the body is positioned within a channel (514) extending distally from an opening (518) in a radially inner surface of the lumen (525);
the channel (514) has a longitudinal axis transverse to the longitudinal axis of the lumen (525), wherein a surface (515) of the body is configured to slidably interface with the channel (514) when the actuator (550) is moved proximally or distally.

7. The device of claim 6, wherein the body is configured to slide proximally within the channel (514), move through the opening (518) in the radially inner surface of the lumen (525), and enter the channel (514), when the actuator (550) is moved proximally.

8. A device comprising:
a shaft (102) having a distal end (701) and a lumen (725), the lumen (725) terminating in a distally-facing opening, wherein an instrument (727) is extendable through the lumen (725) and out of opening; and
an elevator (702) for engaging the instrument (727), the elevator (702) including:
an actuator (750) extending through at least a portion of the shaft (102); and
a body (706) coupled to the actuator (450), wherein a portion of the body (706) is configured to extend within the lumen (725) for selectively positioning the instrument (727),
wherein the elevator (702) further includes:
a tab member (710) pivotally movable relative to lumen (725); and
a block (707) fixedly coupled to the shaft (102) and including a first surface (708) contacting a second surface of the body, wherein the first surface (708) is transverse to the longitudinal axis of the lumen (725); and wherein the second surface is configured to slidably interface with the first surface (708) when the actuator (750) is moved proximally or distally; and
the body (706) is configured to move towards the lumen (725) and force the tab member (710) into the lumen (725).

9. The device of claim 8, wherein the tab member (710) is biased away from the lumen (725).

10. The device of claim 8, wherein the actuator (750) extends through a channel (755) within the block (707).

11. A device comprising:
a shaft (102) having a distal end and a lumen (1225), the lumen (1225) terminating in a distally-facing opening, wherein an instrument (1227) is extendable through the lumen (1225) and out of opening; and
an elevator (1270) for engaging the instrument (1227), the elevator (1270) including:
an actuator (1210) extending through at least a portion of the shaft (102); and
a body (1211) coupled to the actuator (1210), wherein a portion of the body (1211) is configured to extend within the lumen (1225) for selectively positioning the instrument (1227),
wherein the lumen (1225) is a first lumen, and the body (1211) is a snare loop, wherein the elevator (1270) further includes:
a support (1203) including a second lumen (1209) and a channel (1205) extending circumferentially around a radially inner surface of the second lumen (1209), wherein the second lumen (1209) aligns with the first lumen (1225); wherein the channel (1205) receives the snare loop (1211); and
wherein the snare loop (1211) is configured to enter the second lumen (1209) when the actuator (1210) is moved proximally.

12. The device of claim 11, wherein the snare loop (1211) is biased towards the channel (1205).

## Patentansprüche

1. Vorrichtung (104), aufweisend:
einen Schaft (102) mit einem distalen Ende (101, 201) und einem Lumen (225), wobei das Lumen (225) in einer nach distal weisenden Öffnung endet, wobei ein Instrument (227) durch das Lumen (225) und aus der Öffnung führbar ist; und
eine Hebevorrichtung (242) zum Eingriff in das Instrument (227), wobei die Hebevorrichtung (242) aufweist:
einen Aktuator (240), der sich durch mindestens einen Abschnitt des Schafts (102) erstreckt; und
einen Körper (244, 246, 248), der mit dem Aktuator (240) gekoppelt ist, wobei ein Abschnitt des Körpers (244, 246, 248) dazu konfiguriert ist, sich in dem Lumen (225) zu erstrecken, um das Instrument (227) selektiv zu positionieren,
wobei:
der Körper (244, 246, 248) eine erste Verlängerung (244), eine zweite Verlängerung (246) und einen proximalen Abschnitt (248) aufweist, der die erste Verlängerung (244) und die zweite Verlängerung (246) verbindet, wobei die zweite Verlängerung (246) eine U-förmige Körperoberfläche für den Eingriff mit dem Instrument (227) aufweist;
der Aktuator (240) mit einem distalen Abschnitt der ersten Verlängerung (244) gekoppelt ist;
ein Abschnitt der zweiten Verlängerung (246) dazu konfiguriert ist, sich innerhalb des Lumens (225) zu bewegen, wenn der Aktuator (240) in proximaler Richtung bewegt wird;
der Abschnitt der zweiten Verlängerung (246) dazu konfiguriert ist, sich aus dem Lumen herauszubewegen, wenn der Aktuator (240) in distaler Richtung bewegt wird; und
eine äußere Fläche (252) der zweiten Verlängerung (246) dazu konfiguriert ist, mit einer radial inneren Fläche des Lumens (225) zu fluchten oder bündig abzuschließen.

2. Vorrichtung (104) nach Anspruch 1, wobei eine Längsachse der ersten Verlängerung (244) quer zu einer Längsachse der zweiten Verlängerung (246) verläuft.

3. Vorrichtung (104) nach einem der Ansprüche 1-2, wobei sich der Körper um eine Achse dreht, die innerhalb des proximalen Abschnitts (248) positioniert ist, wenn der Aktuator (240) in proximaler oder distaler Richtung bewegt wird.

4. Vorrichtung, aufweisend:
einen Schaft (102) mit einem distalen Ende (101, 201) und einem Lumen (325), wobei das Lumen (325) in einer nach distal weisenden Öffnung endet, wobei ein Instrument (427) durch das Lumen (325) und aus der Öffnung führbar ist; und
eine Hebevorrichtung (302) zum Eingriff in das Instrument (427), wobei die Hebevorrichtung (302) aufweist:
einen Aktuator (450), der sich durch mindestens einen Abschnitt des Schafts (102) erstreckt; und
einen Körper (304, 306, 307, 452), der mit dem Aktuator (450) gekoppelt ist, wobei ein Abschnitt des Körpers (304, 306, 307, 452) dazu konfiguriert ist, sich innerhalb des Lumens (325) zu erstrecken, um das Instrument (427) selektiv zu positionieren,
wobei der Körper (304, 306, 307, 452) eine erste Verlängerung (304), eine zweite Verlängerung (306), einen proximalen Abschnitt (307), der die erste Verlängerung (304) und die zweite Verlängerung (306) verbindet, und ein Schwenkelement (452) aufweist, wobei eine radial innere Fläche der ersten Verlängerung (304) und eine radial innere Fläche der zweiten Verlängerung (306) dazu konfiguriert sind, mit einer radial inneren Fläche des Lumens (325) zu fluchten;
der Aktuator (450) mit dem Schwenkelement (452) gekoppelt ist und eine gekrümmte Fläche des Schwenkelements (452) berührt;
ein Abschnitt der ersten Verlängerung (304) dazu konfiguriert ist, sich innerhalb des Lumens (325) zu bewegen, wenn der Aktuator (450) in proximaler Richtung bewegt wird; und
ein Abschnitt der zweiten Verlängerung (306) dazu konfiguriert ist, sich innerhalb des Lumens zu bewegen, wenn der Aktuator (450) in distaler Richtung bewegt wird.

5. Vorrichtung nach Anspruch 4,
wobei der Aktuator (450) mit dem Schwenkelement (452) an einer Stelle gekoppelt ist, die von der Drehachse des Körpers (304, 306, 307, 452) versetzt ist; und wobei eine Längsachse der ersten Verlängerung (304) und eine Längsachse der zweiten Verlängerung (306) parallel verlaufen.

6. Vorrichtung, aufweisend:
einen Schaft (102) mit einem distalen Ende (501) und einem Lumen (525), wobei das Lumen (525) in einer nach distal weisenden Öffnung endet, wobei ein Instrument (527) durch das Lumen (525) und aus der Öffnung führbar ist; und
eine Hebevorrichtung (510) zum Eingriff in das Instrument (527), wobei die Hebevorrichtung (502) aufweist:
einen Aktuator (550), der sich durch mindestens einen Abschnitt des Schafts (102) erstreckt; und
einen Körper, der mit dem Aktuator (550) gekoppelt ist, wobei ein Abschnitt des Körpers dazu konfiguriert ist, sich in dem Lumen (525) zu erstrecken, um das Instrument (527) selektiv zu positionieren,
der Körper in einem Kanal (514) positioniert ist, der sich distal von einer Öffnung (518) in einer radial inneren Fläche des Lumens (525) erstreckt;
der Kanal (514) eine Längsachse quer zur Längsachse des Lumens (525) aufweist, wobei eine Fläche (515) des Körpers dazu konfiguriert ist, gleitend mit dem Kanal (514) zusammenzuwirken, wenn der Aktuator (550) in proximaler oder distaler Richtung bewegt wird.

7. Vorrichtung nach Anspruch 6,
wobei der Körper dazu konfiguriert ist, in proximaler Richtung innerhalb des Kanals (514) zu gleiten, sich durch die Öffnung (518) in der radial inneren Fläche des Lumens (525) zu bewegen und in den Kanal (514) einzutreten, wenn der Aktuator (550) in proximaler Richtung bewegt wird.

8. Vorrichtung, aufweisend:
einen Schaft (102) mit einem distalen Ende (701) und einem Lumen (725), wobei das Lumen (725) in einer nach distal weisenden Öffnung endet, wobei ein Instrument (727) durch das Lumen (725) und aus der Öffnung führbar ist; und
eine Hebevorrichtung (702) zum Eingriff in das Instrument (727), wobei die Hebevorrichtung (702) aufweist:
einen Aktuator (750), der sich durch mindestens einen Abschnitt des Schafts (102) erstreckt; und
einen Körper (706), der mit dem Aktuator (450) gekoppelt ist, wobei ein Abschnitt des Körpers (706) dazu konfiguriert ist, sich in dem Lumen (725) zu erstrecken, um das Instrument (727) selektiv zu positionieren,
wobei die Hebevorrichtung (702) ferner aufweist:
ein Laschenelement (710), das relativ zum Lumen (725) schwenkbar ist; und
einen Block (707), der fest mit dem Schaft (102) verbunden ist und eine erste Fläche (708) aufweist, die eine zweite Fläche des Körpers berührt, wobei die erste Fläche (708) quer zur Längsachse des Lumens (725) verläuft; und wobei die zweite Fläche dazu konfiguriert ist, gleitend mit der ersten Fläche (708) zusammenzuwirken, wenn der Aktuator (750) in proximaler oder distaler Richtung bewegt wird; und
der Körper (706) dazu konfiguriert ist, sich zum Lumen (725) zu bewegen und das Laschenelement (710) in das Lumen (725) zu drücken.

9. Vorrichtung nach Anspruch 8,
wobei das Laschenelement (710) vom Lumen (725) weg vorgespannt ist.

10. Vorrichtung nach Anspruch 8,
wobei sich der Aktuator (750) durch einen Kanal (755) innerhalb des Blocks (707) erstreckt.

11. Vorrichtung, aufweisend:
einen Schaft (102) mit einem distalen Ende und einem Lumen (1225), wobei das Lumen (1225) in einer nach distal weisenden Öffnung endet, wobei ein Instrument (1227) durch das Lumen (1225) und aus der Öffnung führbar ist; und
eine Hebevorrichtung (1270) zum Eingriff in das Instrument (1227), wobei die Hebevorrichtung (1270) aufweist:
einen Aktuator (1210), der sich durch mindestens einen Abschnitt des Schafts (102) erstreckt; und
einen Körper (1211), der mit dem Aktuator (1210) gekoppelt ist, wobei ein Abschnitt des Körpers (1211) dazu konfiguriert ist, sich in dem Lumen (1225) zu erstrecken, um das Instrument (1227) selektiv zu positionieren,
wobei das Lumen (1225) ein erstes Lumen ist und der Körper (1211) eine Schlingenschlaufe ist, wobei die Hebevorrichtung (1270) ferner aufweist:
ein Tragelement (1203) mit einem zweiten Lumen (1209) und einem Kanal (1205), der sich in Umfangsrichtung um eine radial innere Fläche des zweiten Lumens (1209) erstreckt, wobei das zweite Lumen (1209) mit dem ersten Lumen (1225) fluchtet; wobei der Kanal (1205) die Schlingenschlaufe (1211) aufnimmt; und
wobei die Schlingenschlaufe (1211) dazu konfiguriert ist, in das zweite Lumen (1209) einzutreten, wenn der Aktuator (1210) in proximaler Richtung bewegt wird.

12. Vorrichtung nach Anspruch 11,
wobei die Schlingenschlaufe (1211) zum Kanal (1205) hin vorgespannt ist.

## Revendications

1. Dispositif (104) comprenant :
une tige (102) ayant une extrémité distale (101, 201) et une lumière (225), la lumière (225) se terminant par une ouverture orientée de manière distale, dans lequel un instrument (227) est extensible à travers la lumière (225) et hors de l'ouverture ; et
un élévateur (242) pour mettre en prise l'instrument (227), l'élévateur (242) comprenant :
un actionneur (240) s'étendant à travers au moins une partie de la tige (102) ; et
un corps (244, 246, 248) couplé à l'actionneur (240), dans lequel une partie du corps (244, 246, 248) est configurée pour s'étendre à l'intérieur de la lumière (225) afin de positionner sélectivement l'instrument (227),
dans lequel :
le corps (244, 246, 248) comprend une première extension (244), une deuxième extension (246) et une partie proximale (248) raccordant la première extension (244) et la deuxième extension (246), la deuxième extension (246) ayant une surface de corps en forme de U pour mettre en prise l'instrument (227) ;
l'actionneur (240) est couplé à une partie distale de la première extension (244) ;
une partie de la deuxième extension (246) est configurée pour se déplacer à l'intérieur de la lumière (225) lorsque l'actionneur (240) est déplacé, de manière proximale ;
la partie de la deuxième extension (246) est configurée pour sortir de la lumière lorsque l'actionneur (240) est déplacé de manière distale ; et
une surface extérieure (252) de la deuxième extension (246) est configurée pour s'aligner avec ou être à fleur avec une surface radialement interne de la lumière (225).

2. Dispositif (104) selon la revendication 1, dans lequel un axe longitudinal de la première extension (244) est transversal par rapport à un axe longitudinal de la deuxième extension (246).

3. Dispositif (104) selon l'une quelconque des revendications 1 à 2, dans lequel le corps tourne autour d'un axe positionné dans la partie proximale (248), lorsque l'actionneur (240) est déplacé de manière proximale ou distale.

4. Dispositif comprenant :
une tige (102) ayant une extrémité distale (101, 201) et une lumière (325), la lumière (325) se terminant par une ouverture orientée de manière distale, dans lequel un instrument (427) est extensible à travers la lumière (325) et hors de l'ouverture ; et
un élévateur (302) pour mettre en prise l'instrument (427), l'élévateur (302) comprenant :
un actionneur (450) s'étendant à travers au moins une partie de la tige (102) ; et
un corps (304, 306, 307, 452) couplé à l'actionneur (450), dans lequel une partie du corps (304, 306, 307, 452) est configurée pour s'étendre à l'intérieur de la lumière (325) afin de positionner sélectivement l'instrument (427),
le corps (304, 306, 307, 452) comprend une première extension (304), une deuxième extension (306), une partie proximale (307) raccordant la première extension (304) et la deuxième extension (306), et un élément de pivot (452), une surface radialement interne de la première extension (304) et une surface radialement interne de la deuxième extension (306) étant configurées pour s'aligner avec une surface radialement interne de la lumière (325) ;
l'actionneur (450) est couplé à l'élément de pivot (452) et est en contact avec une surface courbée de l'élément de pivot (452) ;
une partie de la première extension (304) est configurée pour se déplacer à l'intérieur de la lumière (325), lorsque l'actionneur (450) est déplacé de manière proximale ; et
une partie de la deuxième extension (306) est configurée pour se déplacer à l'intérieur de la lumière, lorsque l'actionneur (450) est déplacé de manière distale.

5. Dispositif selon la revendication 4, dans lequel l'actionneur (450) est couplé à l'élément de pivot (452) dans une position décalée par rapport à l'axe de rotation du corps (304, 306, 307, 452) ; et dans lequel un axe longitudinal de la première extension (304) et un axe longitudinal de la deuxième extension (306) sont parallèles.

6. Dispositif comprenant :
une tige (102) ayant une extrémité distale (501) et une lumière (525), la lumière (525) se terminant par une ouverture orientée de manière distale, dans lequel un instrument (527) est extensible à travers la lumière (525) et hors de l'ouverture ; et
un élévateur (510) pour mettre en prise l'instrument (527), l'élévateur (502) comprenant :
un actionneur (550) s'étendant à travers au moins une partie de la tige (102) ; et
un corps couplé à l'actionneur (550), dans lequel une partie du corps est configurée pour s'étendre à l'intérieur de la lumière (525) afin de positionner sélectivement l'instrument (527),
le corps est positionné à l'intérieur d'un canal (514) s'étendant de manière distale à partir d'une ouverture (518) dans une surface radialement interne de la lumière (525) ;
le canal (514) a un axe longitudinal transversal par rapport à l'axe longitudinal de la lumière (525), dans lequel une surface (515) du corps est configurée pour s'interfacer, de manière coulissante, avec le canal (514), lorsque l'actionneur (550) est déplacé de manière proximale ou distale.

7. Dispositif selon la revendication 6, dans lequel le corps est configuré pour coulisser, de manière proximale, à l'intérieur du canal (514), se déplacer à travers l'ouverture (518) dans la surface radialement interne de la lumière (525) et entrer dans le canal (514), lorsque l'actionneur (550) est déplacé de manière proximale.

8. Dispositif comprenant :
une tige (102) ayant une extrémité distale (701) et une lumière (725), la lumière (725) se terminant par une ouverture orientée de manière distale, dans lequel un instrument (727) est extensible à travers la lumière (725) et hors de l'ouverture ; et
un élévateur (702) pour mettre en prise l'instrument (727), l'élévateur (702) comprenant :
un actionneur (750) s'étendant à travers au moins une partie de la tige (102) ; et
un corps (706) couplé à l'actionneur (450), dans lequel une partie du corps (706) est configurée pour s'étendre à l'intérieur de la lumière (725) afin de positionner sélectivement l'instrument (727),
dans lequel l'élévateur (702) comprend en outre :
un élément de languette (710) mobile, de manière pivotante, par rapport à la lumière (725) ; et
un bloc (707) couplé, de manière fixe, à la tige (102) et comprenant une première surface (708) en contact avec une deuxième surface du corps, dans lequel la première surface (708) est transversale par rapport à l'axe longitudinal de la lumière (725) ; et dans lequel la deuxième surface est configurée pour s'interfacer, de manière coulissante, avec la première surface (708), lorsque l'actionneur (750) est déplacé de manière proximale ou distale ; et
le corps (706) est configuré pour se déplacer vers la lumière (725) et forcer l'élément de languette (710) dans la lumière (725).

9. Dispositif selon la revendication 8,
dans lequel l'élément de languette (710) est sollicité à l'opposé de la lumière (725).

10. Dispositif selon la revendication 8,
dans lequel l'actionneur (750) s'étend à travers un canal (755) à l'intérieur du bloc (707).

11. Dispositif comprenant :
une tige (102) ayant une extrémité distale et une lumière (1225), la lumière (1225) se terminant par une ouverture orientée de manière distale, dans lequel un instrument (1227) est extensible à travers la lumière (1225) et hors de l'ouverture ; et
un élévateur (1270) pour mettre en prise l'instrument (1227), l'élévateur (1270) comprenant :
un actionneur (1210) s'étendant à travers au moins une partie de la tige (102) ; et
un corps (1211) couplé à l'actionneur (1210), dans lequel une partie du corps (1211) est configurée pour s'étendre à l'intérieur de la lumière (1225) afin de positionner sélectivement l'instrument (1227),
dans lequel la lumière (1225) est une première lumière, et le corps (1211) est une boucle d'anse, dans lequel l'élévateur (1270) comprend en outre :
un support (1203) comprenant une deuxième lumière (1209) et un canal (1205) s'étendant de manière circonférentielle autour d'une surface radialement interne de la deuxième lumière (1209), dans lequel la deuxième lumière (1209) s'aligne avec la première lumière (1225) ; dans lequel le canal (1205) reçoit la boucle d'anse (1211) ; et
dans lequel la boucle d'anse (1211) est configurée pour entrer dans la deuxième lumière (1209) lorsque l'actionneur (1210) est déplacé de manière proximale.

12. Dispositif selon la revendication 11, dans lequel la boucle d'anse (1211) est sollicitée vers le canal (1205).
